# EUROPEAN PATENT SPECIFICATION

(11) **EP 3 813 750 B1**
(45) Date of publication and mention of the grant of the patent: **02.03.2022**
(21) Application number: 19713623.7
(22) Date of filing: 11.03.2019
(51) Int. Cl.: A61F 13/02

(54) **RELEASE LINER WITH EDGE PROTECTION**
TRENNSCHICHT MIT KANTENSCHUTZ
DOUBLURE DÉTACHABLE DOTÉE D'UNE PROTECTION DE BORD

(30) Priority: 28.06.2018 US 201862691107 P
(43) Date of publication of application: 05.05.2021
(73) Proprietor: KCI Licensing, Inc., San Antonio, TX 78265-9508 (US)
(72) Inventor: REHBEIN, Jonathan G., San Antonio, TX 78232 (US); CAVANAUGH, Matthew Francis, San Antonio, TX 78258 (US)
(74) Representative: Simmons & Simmons
(86) International application number: PCT/US2019/021572
(87) International publication number: WO 2020/005344

(56) References cited:
- WO-A1-2015/130471
- WO-A1-2015/173547
- GB-A- 2 540 955
- US-A1- 2014 227 483

## Description

### TECHNICAL FIELD

The invention set forth in the appended claims relates generally to tissue treatment systems and more particularly, but without limitation, to a drape or cover for treating a tissue site.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

### BRIEF SUMMARY

There is provided a cover for sealing a tissue site, the cover comprising: a shell having a first side, a second side, and an edge between the first side and the second side; an adhesive disposed on the first side; and a release liner adjacent to the adhesive, the release liner comprising a flap folded over at least a portion of the edge of the shell; wherein the shell, the adhesive, and the release liner are folded and the flap is at least partially disposed between a first portion and a second portion of the second side of the shell.

A selection of optional features is set out in the dependent claims.

### BRIEF DESCRIPTION OF THE DRAWINGS

Figure 1 is a functional block diagram of an example embodiment of a therapy system that can provide negative-pressure treatment in accordance with this specification;
Figure 2 is an assembly view of a cover that may be associated with the therapy system of Figure 1, illustrating additional details that may be associated with some embodiments;
Figure 3 is a view of the cover of Figure 2 as assembled;
Figure 4 is a view of the cover of Figure 3, as partially folded; and
Figure 5 is a view of the cover of Figure 3, as folded for packaging.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

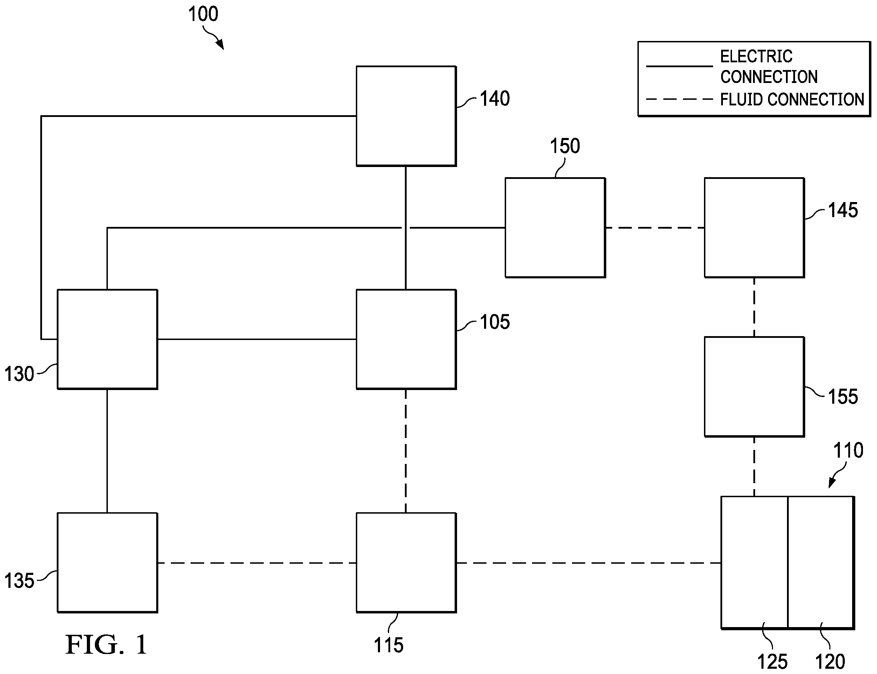

Figure 1 is a simplified functional block diagram of an example embodiment of a therapy system 100 that can provide negative-pressure therapy to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130 and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. For example, the tensile strength of foam may be increased for instillation of topical treatment solutions. The 25% compression load deflection of the tissue interface 120 may be at least 0.35 pounds per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per inch. In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transfer rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

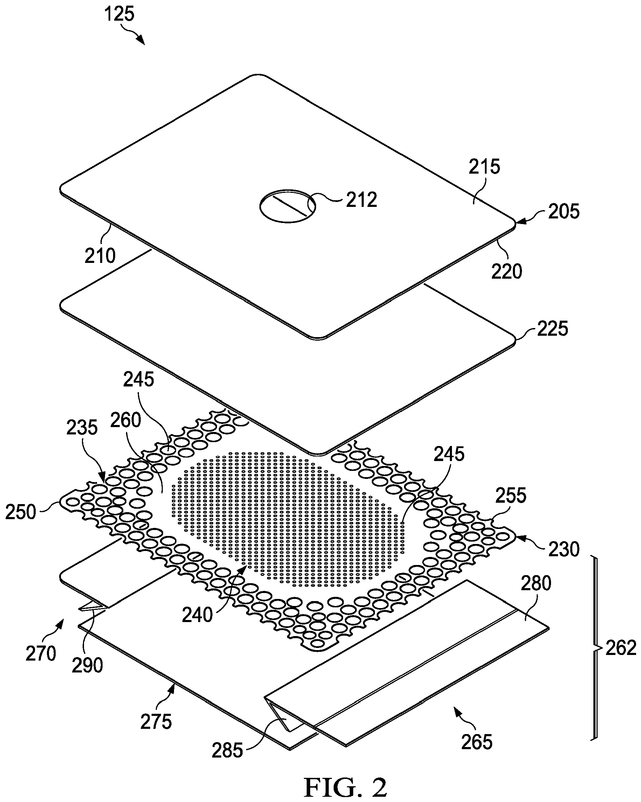

Figure 2 is an assembly diagram of an example of the cover 125, illustrating additional details that may be associated with some embodiments. In the example of Figure 2, the cover 125 comprises a shell 205 having two faces or sides, such as a first side 210 and a second side 215, and an edge 220 between the first side 210 and the second side 215. The shell 205 may also have an aperture 212 configured to receive or be coupled to a fluid conductor. In some examples, the shell 205 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The shell 205 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

The cover 125 may additionally include an attachment device, which may be used to attach the shell 205 to an attachment surface, such as undamaged epidermis, a gasket, or another cover. The attachment device may take many forms. For example, in Figure 2, the attachment device consists essentially of an adhesive 225 disposed on one face or side of the shell 205. In some embodiments, the adhesive 225 may be a coating or layer on one face or side of the shell 205, such as the first side 210. In some examples, the adhesive 225 may be a medically-acceptable, pressure-sensitive adhesive. The adhesive 225 may be an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In the example of Figure 2, the cover 125 additionally comprises a sealing layer 230. In some embodiments, the sealing layer 230 may be adjacent to at least some portion of the adhesive 225 opposite the shell 205. The sealing layer 230 may comprise or consist essentially of a soft, pliable material suitable for providing a fluid seal with a tissue site, and may have a substantially flat surface. For example, the sealing layer 230 may comprise, without limitation, a silicone gel, a soft silicone, hydrocolloid, hydrogel, polyurethane gel, polyolefin gel, hydrogenated styrenic copolymer gel, a foamed gel, a soft closed cell foam such as polyurethanes and polyolefins coated with an adhesive, polyurethane, polyolefin, or hydrogenated styrenic copolymers. In some embodiments, the sealing layer 230 may have a thickness between about 200 microns (µm) and about 1000 microns (µm). In some embodiments, the sealing layer 230 may have a hardness between about 5 Shore OO and about 80 Shore OO. Further, the sealing layer 230 may be comprised of hydrophobic or hydrophilic materials.

In some embodiments, the sealing layer 230 may be a hydrophobic-coated material. For example, the sealing layer 230 may be formed by coating a spaced material, such as, for example, woven, nonwoven, molded, or extruded mesh with a hydrophobic material. The hydrophobic material for the coating may be a soft silicone, for example.

The sealing layer 230 may have a periphery 235 surrounding or around an interior portion 240, and apertures 245 disposed through the periphery 235 and the interior portion 240. The interior portion 240 may correspond to a surface area of the shell 205 in some examples. The sealing layer 230 may also have corners 250 and edges 255. The corners 250 and the edges 255 may be part of the periphery 235. The sealing layer 230 may have an interior border 260 around the interior portion 240, disposed between the interior portion 240 and the periphery 235. The interior border 260 may be substantially free of the apertures 245, as illustrated in the example of Figure 2. In some examples, as illustrated in Figure 2, the interior portion 240 may be symmetrical and centrally disposed in the sealing layer 230.

The apertures 245 may be formed by cutting or by application of local RF or ultrasonic energy, for example, or by other suitable techniques for forming an opening. The apertures 245 may have a uniform distribution pattern, or may be randomly distributed on the sealing layer 230. The apertures 245 in the sealing layer 230 may have many shapes, including circles, squares, stars, ovals, polygons, slits, complex curves, rectilinear shapes, triangles, for example, or may have some combination of such shapes.

Each of the apertures 245 may have uniform or similar geometric properties. For example, in some embodiments, each of the apertures 245 may be circular apertures, having substantially the same diameter. In some embodiments, each of the apertures 245 may have a diameter of about 1 millimeter to about 50 millimeters. In other embodiments, the diameter of each of the apertures 245 may be about 1 millimeter to about 20 millimeters.

In other embodiments, geometric properties of the apertures 245 may vary. For example, the diameter of the apertures 245 may vary depending on the position of the apertures 245 in the sealing layer 230, as illustrated in Figure 2. In some embodiments, the diameter of the apertures 245 in the periphery 235 of the sealing layer 230 may be larger than the diameter of the apertures 245 in the interior portion 240 of the sealing layer 230. For example, in some embodiments, the apertures 245 disposed in the periphery 235 may have a diameter between about 9.8 millimeters and about 10.2 millimeters. In some embodiments, the apertures 245 disposed in the corners 250 may have a diameter between about 7.75 millimeters and about 8.75 millimeters. In some embodiments, the apertures 245 disposed in the interior portion 240 may have a diameter between about 1.8 millimeters and about 2.2 millimeters.

At least one of the apertures 245 in the periphery 235 may be positioned at the edges 255 of the periphery 235, and may have an interior cut open or exposed at the edges 255 that is in fluid communication in a lateral direction with the edges 255. The lateral direction may refer to a direction toward the edges 255 and in the same plane as the sealing layer 230. As shown in the example of Figure 2, the apertures 245 in the periphery 235 may be positioned proximate to or at the edges 255 and in fluid communication in a lateral direction with the edges 255. The apertures 245 positioned proximate to or at the edges 255 may be spaced substantially equidistant around the periphery 235 as shown in the example of Figure 2. Alternatively, the spacing of the apertures 245 proximate to or at the edges 255 may be irregular.

As illustrated in the example of Figure 2, in some embodiments, the cover 125 may include a release liner 262 to protect the adhesive 225 prior to use. The release liner 262 may also provide stiffness to assist with, for example, deployment of the cover 125. The release liner 262 may comprise two or more release panels in some embodiments. For example, the release liner 262 may comprise one or more panels that can be positioned along opposing edges of the sealing layer 230. A first release panel may overlap or otherwise extend over a portion of a second release panel in some embodiments. In other embodiments, the release liner 262 may additionally have a third release panel, which can be overlap or otherwise extend over a portion of at least one of the other release panels. In the example of Figure 2, the release liner 262 comprises a first edge panel 265, a second edge panel 270, and a center panel 275 extending over the first edge panel 265 and the second edge panel 270.

The release liner 262 may additionally include or be coupled to a flap 280. In some embodiments, the flap 280 may be integral to or otherwise coupled to a release panel. In Figure 2, for example, the flap 280 extends from the first edge panel 265.

The release liner 262 may also have one or more release tabs, which may be integral to or otherwise coupled to one or more release panels in some embodiments. As illustrated in Figure 2, a first release tab 285 may be coupled to the first edge panel 265, and a second release tab 290 may be coupled to the second edge panel 270.

The release liner 262 (or one or more release panels) may comprise or consist essentially of a casting paper or a polymer film, for example. In some embodiments, the release liner 262 may comprise or consist of a polyethylene film. Further, in some embodiments, the release liner 262 may be a polyester material such as polyethylene terephthalate (PET), or similar polar semi-crystalline polymer. The use of a polar semi-crystalline polymer for the release liner 262 may substantially preclude wrinkling or other deformation of the cover 125. For example, the polar semi-crystalline polymer may be highly orientated and resistant to softening, swelling, or other deformation that may occur when brought into contact with components of the cover 125, or when subjected to temperature or environmental variations, or sterilization. Further, a release agent may be disposed on a side of the release liner 262 that is configured to contact the sealing layer 230. For example, the release agent may be a silicone coating and may have a release factor suitable to facilitate removal of the release liner 262 by hand and without damaging or deforming the cover 125. In some embodiments, the release agent may be a fluorocarbon or a fluorosilicone, for example. In other embodiments, the release liner 262 may be uncoated or otherwise used without a release agent.

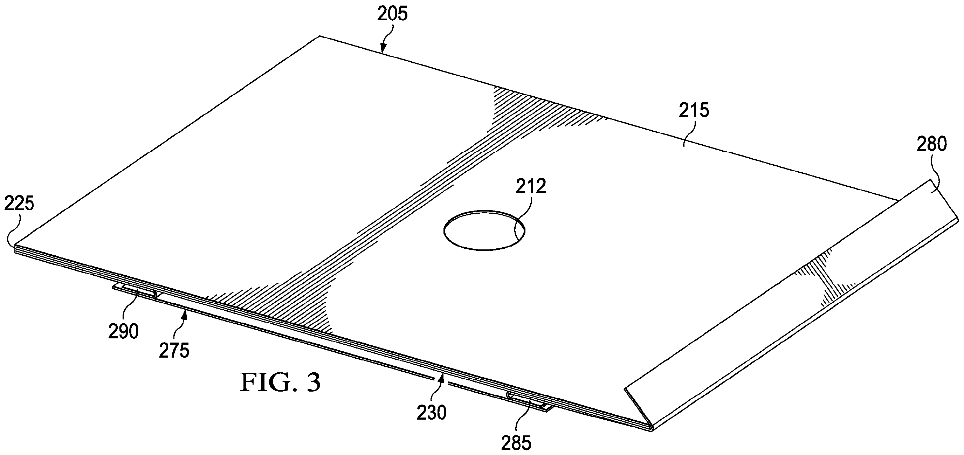

Figure 3 is a perspective view of the cover 125 of Figure 2 as assembled, illustrating additional details that may be associated with some embodiments. In some examples, the flap 280 may be folded over at least a portion of the one or more edges of the shell 205, which can substantially reduce or prevent the adhesive 225 from migrating past the edges. As illustrated in the example of Figure 3, the flap 280 may be folded over the edge 220 (not visible). The center panel 275 may extend over the first release tab 285, the second release tab 290, or both in some examples.

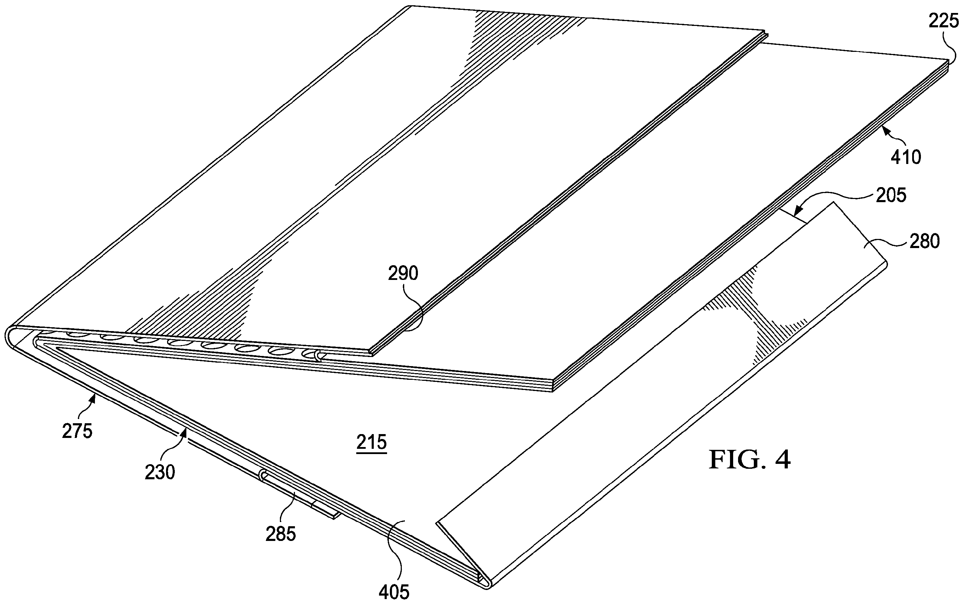

Figure 4 is a perspective view of the cover 125 of Figure 3 in a partially folded configuration, illustrating additional details that may be associated with some embodiments. In the example of Figure 4, the shell 205, the adhesive 225, and the release liner 262 are doubled over, and the flap 280 is at least partially disposed between a first portion 405 and a second portion 410 of the shell 205. In some embodiments, the shell 205, the adhesive 225, and the release liner 262 may be folded approximately in half, and the first portion 405 may be substantially the same size as the second portion 410 to minimize the dimensions of the folded configuration.

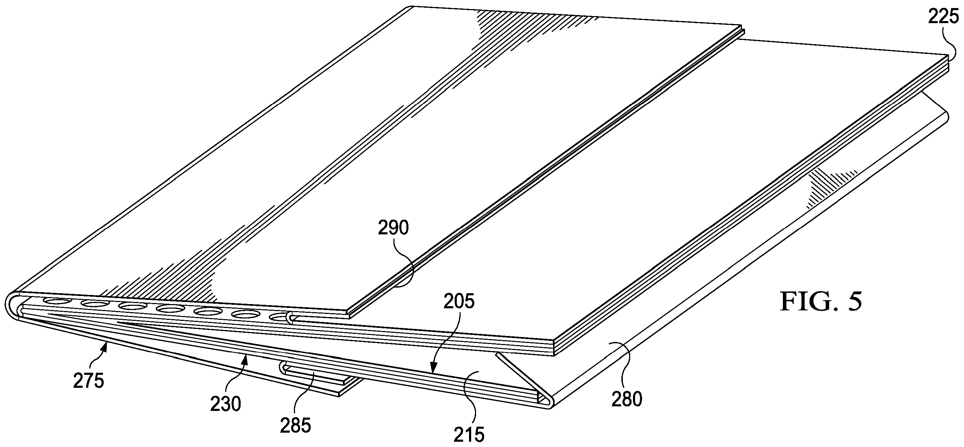

Figure 5 is a perspective view of the cover 125 of Figure 4 in a substantially folded configuration. The example configuration of Figure 5 may be advantageous for packaging and storing some embodiments of the cover 125. For example, the folded configuration of 125 may be particularly suitable for storing in a pouch or other container, and the flap 280 can reduce or prevent the adhesive 225 from sticking to the container and other parts of the cover 125, including other portions of the shell 205.

In use, the cover 125 may be removed from a package and poured into a sterile environment. If folded, the cover 125 may be unfolded, and the flap 280 may also be unfolded. The release liner 262 may be removed to expose the sealing layer 230, which may be placed within, over, on, or otherwise proximate to a tissue site. For example, the center panel 275 may be removed and a center portion of the sealing layer 230 may be placed over a surface tissue site and adjacent epidermis. The first edge panel 265 may be removed by pulling the first release tab 285, the second edge panel 270 may be removed by pulling the second release tab 290, and the edge portions of the sealing layer 230 may be applied to the adjacent epidermis. In some applications, the tissue interface 120 may be placed within, over, on, or otherwise proximate to a tissue site, and the sealing layer may be placed over the tissue interface 120. The interior portion 240 of the sealing layer 230 may be positioned adjacent to, proximate to, or covering a tissue site. The periphery 235 of the sealing layer 230 may be positioned adjacent to or proximate to tissue around or surrounding the tissue site. The sealing layer 230 may be sufficiently tacky to hold the cover 125 in position, while also allowing the cover 125 to be removed or re-positioned without trauma to the tissue site.

Removing the release liner 262 can also expose the adhesive 225, and the cover 125 may be attached to an attachment surface, such as epidermis peripheral to a tissue site. For example, the adhesive 225 may be in fluid communication with an attachment surface through the apertures 245 in at least the periphery 235 of the sealing layer 230. The adhesive 225 may also be in fluid communication with the edges 255 through the apertures 245 exposed at the edges 255.

Once the cover 125 is in a desired position, the adhesive 225 may be pressed through the apertures 245 to bond the cover 125 to the attachment surface. The apertures 245 at the edges 255 may permit the adhesive 225 to flow around the edges 255 for enhancing the adhesion of the edges 255 to an attachment surface.

In some embodiments, apertures or holes in the sealing layer may be sized to control the amount of the adhesive 225 exposed through the sealing layer 230. For a given geometry of the corners 250, the relative sizes of the apertures 245 may be configured to maximize the surface area of the adhesive 225 exposed and in fluid communication through the apertures 245 at the corners 250. For example, as shown in Figure 2, the edges 255 may intersect at substantially a right angle, or about 90 degrees, to define the corners 250. In some embodiments, the corners 250 may have a radius of about 10 millimeters. Further, in some embodiments, three of the apertures 245 having a diameter between about 7.75 millimeters to about 8.75 millimeters may be positioned in a triangular configuration at the corners 250 to maximize the exposed surface area for the adhesive 225. In other embodiments, the size and number of the apertures 245 in the corners 250 may be adjusted as appropriate, depending on the chosen geometry of the corners 250, to maximize the exposed surface area of the adhesive 225. Further, the apertures 245 at the corners 250 may be fully contained within the sealing layer 230, substantially precluding fluid communication in a lateral direction exterior to the corners 250. The apertures 245 at the corners 250 being fully contained within the sealing layer 230 may substantially preclude fluid communication of the adhesive 225 exterior to the corners 250, and may provide improved handling of the cover 125 during deployment at a tissue site. Further, the exterior of the corners 250 being substantially free of the adhesive 225 may increase the flexibility of the corners 250 to enhance comfort.

In some embodiments, the bond strength of the adhesive 225 may vary in different locations of the cover 125. For example, the adhesive 225 may have a lower bond strength in locations adjacent to the apertures 245 that are relatively larger, and may have a higher bond strength where the apertures 245 are smaller. Adhesive with lower bond strength in combination with larger apertures may provide a bond comparable to adhesive with higher bond strength in locations having smaller apertures.

Thus, the cover 125 can provide a sealed therapeutic environment proximate to a tissue site, substantially isolated from the external environment. In some applications, a negative-pressure source 105 can reduce the pressure in the sealed therapeutic environment. The sealing layer 230 may provide an effective and reliable seal against challenging anatomical surfaces, such as an elbow or heel, at and around a tissue site. Further, in some embodiments, the cover 125 may re-applied or re-positioned to correct air leaks caused by creases and other discontinuities in the cover 125 or a tissue site, for example. The ability to rectify leaks may increase the efficacy of the therapy and reduce power consumption in some embodiments.

If used with a negative-pressure treatment, the negative-pressure source 105 may be fluidly coupled to a tissue site through the shell 205. For example, if not already configured, a dressing interface may be disposed over the aperture 212 and attached to the shell 205. A fluid conductor may be fluidly coupled to the dressing interface and to the negative-pressure source 105. In other embodiments, a fluid conductor may be inserted directly through the aperture 212, or may be inserted through the shell 205 if the shell 205 does not have an aperture.

The systems, apparatuses, and methods described herein may provide significant advantages. For example, the flap 280 can prevent or substantially reduce migration of the adhesive 225, which can prevent sticking to other parts of the cover 125, packaging, or other objects. In a surgical environment, this can improve handling, which can reduce delays and other complications. In some embodiments, the flap 280 can allow the cover 125 to be folded to reduce size and cost of packaging while mitigating undesirable sticking to the packaging.

Some embodiments of the cover 125 may be particularly advantageous for treating wounds with negative pressure, but the cover 125 may also be beneficial for other treatments. Additionally or alternatively, the cover 125 may be combined with the tissue interface 120 or other treatment elements in some configurations. For example, an absorbent, manifold, or other treatment element may be disposed between portions of the shell 205 or the adhesive 225 and the sealing layer 230 in some embodiments. In some examples, the sealing layer 230 may be omitted.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be eliminated or separated from other components for manufacture or sale. In other example configurations, the controller 130 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims.

## Claims

1. A cover for sealing a tissue site, the cover comprising:
a shell (205) having a first side (210), a second side (215), and an edge between the first side (210) and the second side (215);
an adhesive (225) disposed on the first side (210); and
a release liner (262) adjacent to the adhesive (225), the release liner (262) comprising a flap (280) folded over at least a portion of the edge of the shell (205);
**characterized in that** the shell (205), the adhesive (225), and the release liner (262) are folded and the flap (280) is at least partially disposed between a first portion (405) and a second portion (410) of the second side (215) of the shell (205).

2. The cover of claim 1, wherein:
the release liner (262) comprises a first release panel (265) and a second release panel (270. 275); and
the flap (280) extends from the first release panel (265).

3. The cover of claim 1, further comprising a release tab (285) coupled to the first release panel (265).

4. The cover of claim 3, wherein the second release panel (275) extends over the release tab (285).

5. The cover of claim 1, wherein:
the release liner (262) comprises:
a first edge panel (265) having a first release tab (285),
a second edge panel (270) having a second release tab (290), and
a center panel (275) extending over the first release tab (285) and the second release tab (290); and
the flap (280) extends from the first edge panel (265).

6. The cover of any preceding claim, wherein the shell (205) comprises a polymer that is impermeable to liquid.

7. The cover of any preceding claim, wherein the shell (205) comprises a polymer that is elastomeric.

8. The cover of any preceding claim, wherein the shell (205) comprises polyurethane.

9. The cover of any preceding claim, wherein the adhesive (225) is an acrylic adhesive.

10. The cover of any preceding claim, wherein the adhesive (225) is an acrylic adhesive having a coating weight in a range of about 25-65 grams per square meter.

11. The cover of any preceding claim, further comprising a sealing layer (230) with apertures (245) disposed between the adhesive (225) and the release liner (262).

12. The cover of claim 11, wherein the adhesive (225) is exposed through at least some of the apertures (245).

13. The cover of claim 11 or claim 12, wherein the sealing layer (230) comprises silicone gel.

14. A dressing for treating a tissue site with negative pressure, the dressing comprising:
the cover of any preceding claim; and
a manifold disposed between the adhesive (225) and the release liner (262).

15. A dressing for treating a tissue site with negative pressure, the dressing comprising:
the cover of any one of claims 11-13; and
a manifold disposed between the adhesive (225) and the sealing layer (230).

## Patentansprüche

1. Abdeckung zum Abdichten einer Gewebestelle, wobei die Abdeckung umfasst:
eine Schale (205), die eine erste Seite (210), eine zweite Seite (215) und eine Kante zwischen der ersten Seite (210) und der zweiten Seite (215) aufweist;
einen Kleber (225), der auf der ersten Seite (210) angeordnet ist; und
einen Abziehliner (262), der an den Kleber (225) angrenzt, wobei der Abziehliner (262) eine Klappe (280) umfasst, die über mindestens einen Abschnitt der Kante der Schale (205) gefaltet ist;
**dadurch gekennzeichnet, dass** die Schale (205), der Kleber (225) und der Abziehliner (262) gefaltet sind, und die Klappe (280) mindestens teilweise zwischen einem ersten Abschnitt (405) und einem zweiten Abschnitt (410) der zweiten Seite (215) der Schale (205) angeordnet ist.

2. Abdeckung nach Anspruch 1, wobei:
der Abziehliner (262) eine erste Abziehbahn (265) und eine zweite Abziehbahn (270, 275) umfasst; und
sich die Klappe (280) von der ersten Abziehbahn (265) erstreckt.

3. Abdeckung nach Anspruch 1, die weiter eine Abziehlasche (285), die mit der ersten Abziehbahn (265) gekoppelt ist, umfasst.

4. Abdeckung nach Anspruch 3, wobei sich die zweite Abziehbahn (275) über der Abziehlasche (285) erstreckt.

5. Abdeckung nach Anspruch 1, wobei:
der Abziehliner (262) umfasst:
eine erste Kantenbahn (265), die eine erste Abziehlasche (285) aufweist,
eine zweite Kantenbahn (270), die eine zweite Abziehlasche (290) aufweist, und
eine Mittenbahn (275), die sich über die erste Abziehlasche (285) und die zweite Abziehlasche (290) erstreckt; und
sich die Klappe (280) von der ersten Kantenbahn (265) erstreckt.

6. Abdeckung nach einem vorstehenden Anspruch, wobei die Schale (205) ein Polymer, das flüssigkeitsdicht ist, umfasst.

7. Abdeckung nach einem vorstehenden Anspruch, wobei die Schale (205) ein Polymer, das elastomer ist, umfasst.

8. Abdeckung nach einem vorstehenden Anspruch, wobei die Schale (205) Polyurethan umfasst.

9. Abdeckung nach einem vorstehenden Anspruch, wobei der Kleber (225) ein Acrylkleber ist.

10. Abdeckung nach einem vorstehenden Anspruch, wobei der Kleber (225) ein Acrylkleber ist, der ein Beschichtungsgewicht in einem Bereich von etwa 25 bis 65 Gramm pro Quadratmeter aufweist.

11. Abdeckung nach einem vorstehenden Anspruch, die weiter eine Abdichtungsschicht (230) mit Öffnungen (245), die zwischen dem Kleber (225) und dem Abziehliner (262) angeordnet sind, umfasst.

12. Abdeckung nach Anspruch 11, wobei der Kleber (225) durch mindestens einige der Öffnungen (245) freigelegt ist.

13. Abdeckung nach Anspruch 11 oder Anspruch 12, wobei die Abdichtungsschicht (230) Silikongel umfasst.

14. Verband zum Behandeln einer Gewebestelle mit Unterdruck, wobei der Verband umfasst: die Abdeckung nach einem vorstehenden Anspruch; und
einen Verteiler, der zwischen dem Kleber (225) und dem Abziehliner (262) angeordnet ist.

15. Verband zum Behandeln einer Gewebestelle mit Unterdruck, wobei der Verband umfasst:
die Abdeckung nach einem der Ansprüche 11 bis 13; und
einen Verteiler, der zwischen dem Kleber (225) und der Abdichtungsschicht (230) angeordnet ist.

## Revendications

1. Protection pour isoler un site tissulaire, la protection comprenant :
une enveloppe (205) présentant un premier côté (210), un second côté (215), et un bord entre le premier côté (210) et le second côté (215) ;
un adhésif (225) disposé sur le premier côté (210) ; et
une pellicule détachable (262) adjacente à l'adhésif (225), la pellicule détachable (262) comprenant un rabat (280) replié sur au moins une portion du bord de l'enveloppe (205) ;
**caractérisée en ce que** l'enveloppe (205), l'adhésif (225), et la pellicule détachable (262) sont pliés et le rabat (280) est au moins partiellement disposé entre une première portion (405) et une seconde portion (410) du second côté (215) de l'enveloppe (205).

2. Protection selon la revendication 1, dans laquelle :
la pellicule détachable (262) comprend un premier panneau détachable (265) et un second panneau détachable (270. 275) ; et
le rabat (280) s'étend depuis le premier panneau détachable (265).

3. Protection selon la revendication 1, comprenant en outre une languette détachable (285) couplée au premier panneau détachable (265).

4. Protection selon la revendication 3, dans laquelle le second panneau détachable (275) s'étend sur la languette détachable (285).

5. Protection selon la revendication 1, dans laquelle :
la pellicule détachable (262) comprend :
un premier panneau de bord (265) présentant une première languette détachable (285),
un second panneau de bord (270) présentant une seconde languette détachable (290), et
un panneau central (275) s'étendant sur la première languette détachable (285) et la seconde languette détachable (290) ; et
le rabat (280) s'étend depuis le premier panneau de bord (265).

6. Protection selon une quelconque revendication précédente, dans laquelle l'enveloppe (205) comprend un polymère qui est imperméable au liquide.

7. Protection selon une quelconque revendication précédente, dans laquelle l'enveloppe (205) comprend un polymère qui est élastomère.

8. Protection selon une quelconque revendication précédente, dans laquelle l'enveloppe (205) comprend du polyuréthane.

9. Protection selon une quelconque revendication précédente, dans laquelle l'adhésif (225) est un adhésif acrylique.

10. Protection selon une quelconque revendication précédente, dans laquelle l'adhésif (225) est un adhésif acrylique présentant un poids de couche dans une plage d'environ 25-65 grammes par mètre carré.

11. Protection selon une quelconque revendication précédente, comprenant en outre une couche d'étanchéité (230) avec des ouvertures (245) disposée entre l'adhésif (225) et la pellicule détachable (262).

12. Protection selon la revendication 11, dans laquelle l'adhésif (225) est exposé à travers au moins certaines des ouvertures (245).

13. Protection selon la revendication 11 ou la revendication 12, dans laquelle la couche d'étanchéité (230) comprend un gel de silicone.

14. Pansement destiné à traiter un site tissulaire par pression négative, le pansement comprenant : la protection selon une quelconque revendication précédente ; et
un collecteur disposé entre l'adhésif (225) et la pellicule détachable (262).

15. Pansement destiné à traiter un site tissulaire par pression négative, le pansement comprenant:
la protection selon une quelconque des revendications 11-13 ; et
un collecteur disposé entre l'adhésif (225) et la couche d'étanchéité (230).
